# EUROPEAN PATENT SPECIFICATION

(11) **EP 0 499 188 B1**
(45) Date of publication and mention of the grant of the patent: **09.10.1996**
(21) Application number: 92102218.2
(22) Date of filing: 10.02.1992
(51) Int. Cl.: C07D 263/04, C07D 413/14, C08G 18/32, C08G 18/38, C09K 3/10, C09D 7/12, C09J 11/06

(54) **Polyoxazolidines with carbonate groups, process for their preparation and use thereof**
Polyoxazolidine mit Carbonat-Gruppen, Verfahren zu ihrer Herstellung und ihre Anwendung
Polyoxazolidines à groupes carbonates procédé de préparation et utilisation

(30) Priority: 11.02.1991 IT MI910345
(43) Date of publication of application: 19.08.1992
(73) Proprietor: ENICHEM S.p.A., I-20124 Milano (IT)
(72) Inventor: Greco, Alberto, Dr., I-20077 Dresano, Milan (IT)
(74) Representative: Weinhold, Peter, Dr.

(56) References cited:
- EP-A- 0 087 659
- EP-A- 0 228 935
- EP-A- 0 387 926
- FR-A- 2 359 161
- US-A- 3 864 335

## Description

The present invention relates to new polyoxazolidines, the process for their preparation and their use as cross-linking agents for water-curing systems, particularly those based on polyisocyanates, acrylate polymers and polyepoxides in paint, coating, sealant and adhesive compositions.

B. Taub and G.H. Petschke, in "Polyfunctional Isocyanate-oxazolidine Resins", presented at the Water-Borne and Higher-Solids Coating Symposium, Feb. 1-3, 1989, New Orleans, describe storage-stable one-component urethane coatings which are prepared by reacting aliphatic diisocyanate prepolymers with N-2-hydroxyethyl oxazolidines.

US-A-3,743,626 describes the use of certain polyoxazolidines as curing agents under normal temperature and moisture conditions, for adhesives based on aromatic and aliphatic polyisocyanates. Examples of the polyoxazolidines are bis-(N-ethyl-oxazolidine)carbonates. As described in US-A-4,138,545, these polyoxazolidines can be obtained by means of a reaction of an oxazolidine of formula (A): 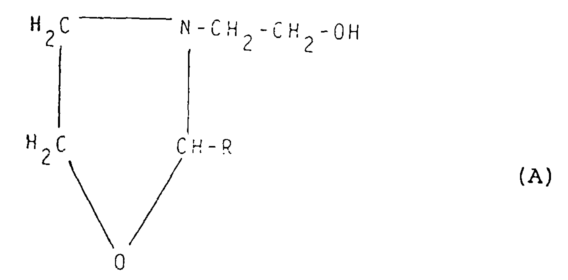 with lower alkyl ethers of di- or polycarboxylic acids, by transesterification or by means of a reaction of an oxazolidine of formula (B): 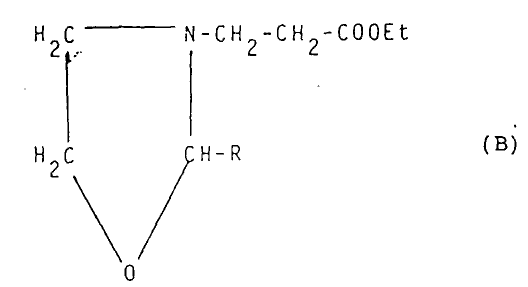 with a glycol or polyol, under transesterification conditions. The oxazolidines (B) are in turn obtained by adding aldehydes to an addition product of ethanolamine and an alkyl acrylate.

These transesterification reactions are generally carried out in the presence of catalysts and the polyoxazolidines thus obtained have ester groups which can easily undergo hydrolysis.

BE-A-856,893 describes the use of certain polyoxazolidines in sealant compositions based on polyisocyanates. As described in BE-A-833,821, these polyoxazolidines can be obtained by the addition of the oxazolidines (A) to polyisocyanates. These products show the disadvantages of high cost and high toxicity due to the polyisocyanates present. In addition, owing to the formation of urethanes during their synthesis, the viscosity of these products is too high, especially when non-aliphatic diisocyanates or polyisocyanates in general are used.

EP-A-228935 describes the use of polyoxazolidines as cross-linking agents in sealing compounds based on polyisocyanates, cross-linkable under normal moisture conditions. These polyoxazolidines require bis-alkanolamines of formula (C) as starting materials:

HO-CH₂-CH₂-NH-(CH₂)ₙ-NH-CH₂-CH₂-OH (C)

The synthesis of these alkanolamines, starting from amines and ehtylene oxide, is not very selective. It is also necessary to separate the corresponding reaction products under high temperature and vacuum from the reaction mass. Distillation is necessary to eliminate the tertiary amines (tri- and polyalkanolamines) which, if introduced into the polyisocyanate systems, reduce the pot life thereof due to premature cross-linking, both chemically (alkanols) and catalytically (presence of tertiary nitrogen).

US-A-4,296,225 describes the use of polyoxazolidines as latent cross-linking agents for polyvinyl systems in the preparation of polyvinylic emulsions. In this case, the oxazolidine is used in the form of a hydroxyalkyl oxazolidine methacrylate, or as component of polyurethane paints having a high solids content. The underlying principle is the insertion of the oxazolidine nucleus into a polyacrylate, which is made possible by using a vinyl oxazolidine capable of copolymerizing to a varying degree with the acrylic monomers. In any case, the oxazolidine equivalent is not high and the polymers obtained are excessively viscous liquids or solids, making it necessary to disperse them in water or dissolve them in an organic solvent.

These drawbacks of the known art have been overcome by the invention disclosed in IT-A-19089A/89, i.e., a group of polyoxazolidines which can be prepared both easily and practically and which can be used as cross-linking agents in water-curing systems based on polyisocyanates, acrylate polymers or polyepoxides.

The only disadvantage of these products is the presence therein of one or more thioether bridges which although imparting stability to photo-oxidation and better oil resistance to the systems into which these products are incorporated, generally give rise to an unpleasant odour typical of polysulphides.

The present invention relates to a new group of polyoxazolidines which overcomes the disadvantages of the known art mentioned above. In particular, a new group of polyoxazolidines has been found, which can be prepared both easily and practically and can be used to particular advantage as cross-linking agents in hygrocuring (water-curing) systems based on polyisocyanates, acrylates or polyepoxides when high fluidity in the liquid state at normal temperature together with a good chemical, thermal and aging resistance is required as in the case of paints, sealants and adhesives. They also endow the final products with an excellent resistance to hydrolysis.

In accordance with this, the first aspect of the present invention relates to new polyoxazolidines corresponding to the general formula (I): 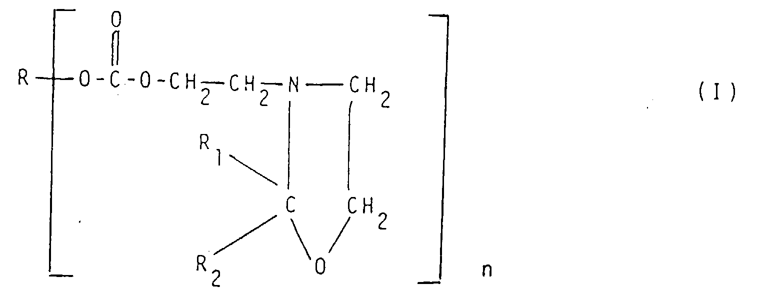 wherein
n ranges from 2 to 10 (e.g. from 2 to 6);
R is an n-valent radical selected from:
   the trivalent triethylisocyanurate group of formula 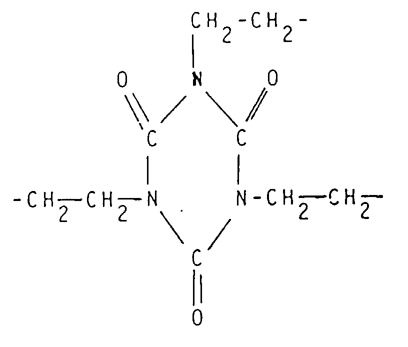 n-valent linear or branched C₂-C₁₂ (e.g. C₂-C₆) alkylene groups, C₅-C₁₂ (e.g. C₅-C₈) cycloalkylene groups, polyethyleneglycol groups of formula -(CH₂-CH₂-O)ₘ-CH₂-CH₂-, m ranging from 1 to 4 (being e.g. 1, 2 or 3), the para-dimethylene cyclohexane radical of formula 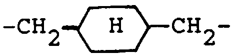 and n-valent groups of general formula: 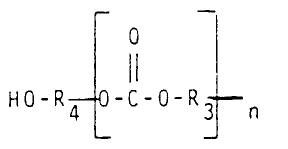
where R₃ is the linear, cyclic or aromatic hydrocarbon residue of a glycol containing up to 12 carbon atoms (e.g. up to 8 carbon atoms) and R₄ has the same meanings as R, excluding R = N-ethyl-oxazolidine group;
R₁ and R₂, the same or different from each other, represent hydrogen, linear or branched C₁-C₆ (e.g. C₁-C₃) alkyl, (preferably C₅-C₈) cycloalkyl or aryl (e.g. phenyl) or R₁ and R₂, together with the carbon atom to which they are linked, represent a saturated C₅-C₇ cycloalkyl ring.

For the purposes of the present invention, the term "aryl" refers to optionally substituted mono-, bi- and tricyclic aromatic radicals containing from 6 to 14 (preferably 6 to 10) carbon atoms.

According to a preferred embodiment, R represents a linear or branched C₂-C₆ bi-, tri- or tetra-valent alkylene group (e.g. ethylene, propylene, butylene), a group of formula 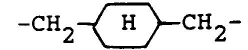 or a cycloalkylene group.

R₁ and R₂ preferably represent independently a linear or branched C₁-C₄ alkyl group (e.g. methyl and ethyl) or one of R₁ and R₂ is hydrogen and the other one is an alkyl group as just mentioned.

Typcial examples of bivalent alkylene or cycloalkylene groups are those of the following formulae:

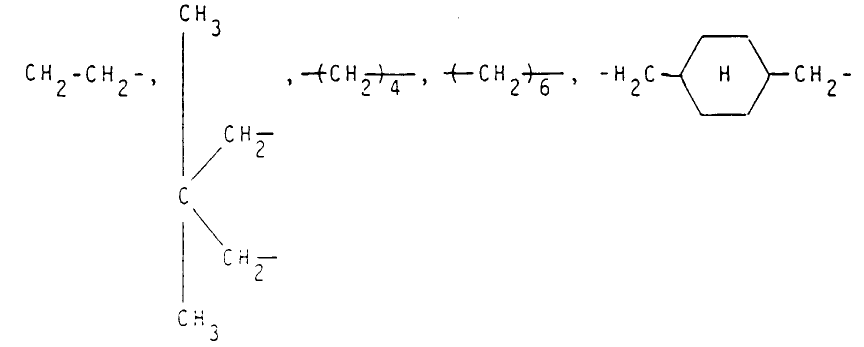

Typcial examples of trivalent alkylene groups are those of the following formulae: 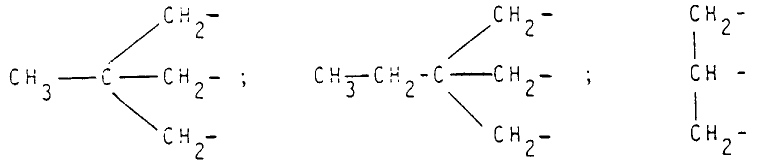 whereas a typical example of a tetravalent alkylene group is that of formula

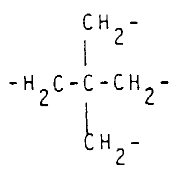

The polyoxazolidines in accordance with the present invention may be obtained as illustrated in the following reaction schemes where, in the formulae, R, R₁, R₂, and n have the meaning specified above.

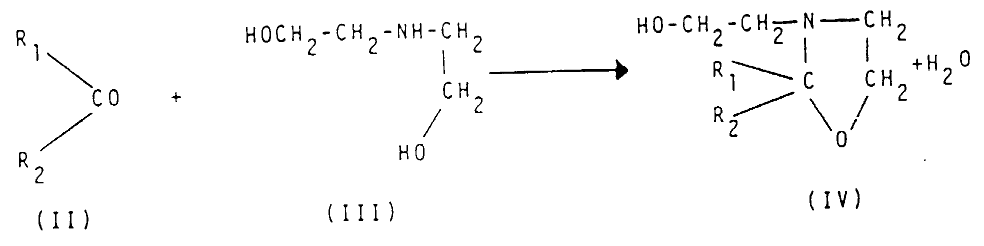

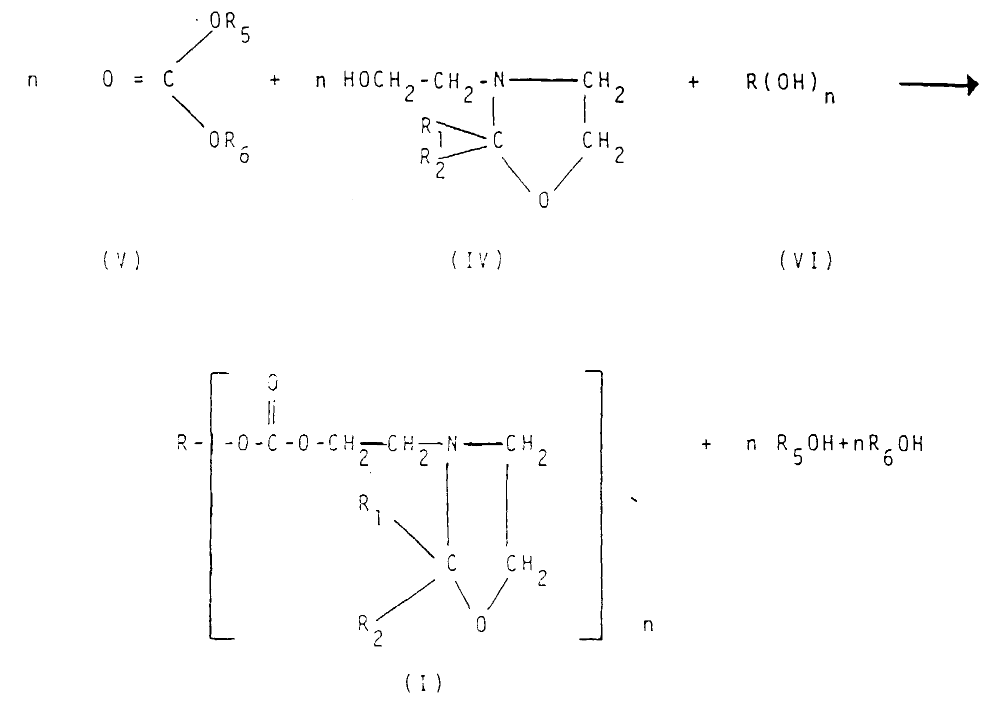

More specifically, with reference to the above scheme, diethanolamine (III) is reacted with an aldehyde or ketone (II) (depending on whether at least one of R₁ and R₂ represents hydrogen or not) to afford N-hydroxyethyl oxazolidine (IV). Specific examples of aldehydes and ketones (II) which can be used for said purpose are formaldehyde, isobutyraldehyde, acetone, methyl ethyl ketone, cyclopentanone and cyclohexanone. It is particularly preferred to use isobutyraldehyde or methyl ethyl ketone.

The reaction between compounds (II) and (III), which his known in the art as is the hydroxyethyl oxazolidine (IV) obtained thereby, is preferably carried out at a temperature ranging from 20 to 160°C and without solvent, by refluxing the aldehyde or ketone so as to azeotropically eliminate the water which is formed as a side product of the reaction. In this case, the aldehyde or ketone is preferably used in an excess of 20 to 100% of the stoichiometric amount and/or in the presence of a chemical dehydrator.

Alternatively, an aliphatic or aromatic solvent can be used, which is capable of forming an azeotropic mixture with water. In this case, a slight excess of the aldehyde or ketone with respect to the stoichiometric amount is preferably used, generally of from 5 to 20%. The reaction is moderately exothermic and takes about 4 to 10 hours, depending on the temperature. At the end of the reaction, the solvent, if any, and excess aldehyde or ketone are eliminated.

The hydroxyethyl oxazolidines can be used as such or, preferably and only when this is possible, they can be purified by distillation under a vacuum of from 0.1 - 1 mm Hg at temperatures ranging from 50 to 150°C, depending on the exact nature of R₁ and R₂.

To facilitate the reaction, a small quantity of an acid catalyst can be added to promote the condensation, such as paratoluene sulfonic acid, sulfonate resins etc. By operating under the above conditions practically quantitative yields of the product may be obtained.

The subsequent transesterification reaction is carried out with hydroxyethyl oxazolidine (IV), a polyol (VI) where R has the meaning specified above, and a carbonate (V) where R₅ and R₆ preferably represent a saturated or unsaturated C₁-C₄ hydrocarbon radical or a (preferably C₆-c₁₀) aryl group (e.g. phenyl), or R₅ and R₆ together with the two adjacent oxygen atoms and the carbonyl carbon atom, form a cyclic carbonate of formula 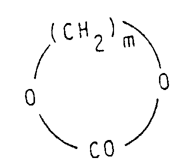 where m is 2 or 3.

Preferred carbonates (V) are those of the following formulae

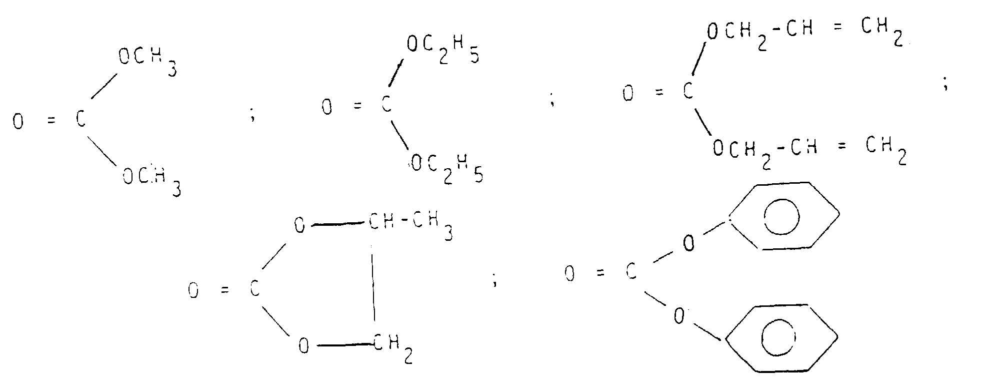

Among the preferred polyvalent alcohols (VI) are di-, tri- and tetra-ethyleneglycol, 1,4-dihydroxymethylcyclohexane, 1,4-butanediol, 2,2-diethyl-butane-1,4-diol, 2,2-dimethyl-1,3-propanediol, glycerol, trimethylol propane, trihydroxyethylisocyanurate and pentaerythritol.

The transesterification reaction is generally carried out at temperatures ranging from 60 to 160°C and preferably from 100 to 140°C, usually in the presence of a transesterification catalyst. The above temperatures are sufficient to remove alcohols R₅OH and R₆OH which are distilled off as soon as they are formed.

In this case the transesterification reaction usually is carried out at atmospheric pressure. Alternatively, it also is possible to operate at a partially reduced pressure to facilitate the removal of the alcohols R₅OH and R₆OH.

The transesterification reaction does not require strict stoichiometric amounts and preferably is even carried out with an excess of hydroxyethyl oxazolidine (IV) of up to 30% of the stoichiometric quantity. Upon completion of the transesterification reaction the excess of hydroxyethyl oxazolidine (IV) may then be removed by distillation, optionally under reduced pressure.

If the transesterification reaction is carried out with a not very volatile oxazolidine (e.g. R₁ and R₂ representing particularly heavy groups), it is preferable to employ an excess of carbonate (V) instead of an excess of hydroxyethyl oxazolidine (IV) the unreacted portion whereof would be difficult to remove at the end of the reaction. In this case, the unreacted carbonate may be removed by distillation under reduced pressure at the end of the reaction. When the reaction is complete and after the removal of any possible residual quantities of hydroxyethyl oxazolidine and/or carbonate by distillation, the product obtained is cooled and the catalyst eliminated, using the most suitable techniques for the catalytic system used.

Examples of transesterification catalysts which can be used are alkali metals, sodium and potassium alcoholates of low-boiling alcohols and organometallic compounds of Sn(IV), Ti(IV) or other metals; preferably sodium methylate is used.

The quantity of catalyst used generally ranges from 50 to 1000 ppm with respect to the mixture of reactants.

If sodium methylate is used as catalyst, its removal at the end of the reaction may involve neutralization with an organic or inorganic acid in a stoichiometric quantity or preferably in slight excess (e.g. 50-30%) with respect to the sodium methylate. Any free acidity may be removed by treatment with calcium oxide and subsequent filtration.

The polyoxazolidine (I) may be obtained in almost quantitative yield.

The advantage of polyoxazolidines of the carbonate type corresponding to formula (I) compared to those of the polyester type of the known art is that the final products have a higher hydrolytic resistance, as evidenced by the examples below.

The process for the preparation of the polyoxazolidines (I) in accordance with the present invention has various advantages. The main advantage is that it affords a high yield and selectivity of the reactions involved. In addition, stable and easily processable intermediates may be used together with catalysts which are not harmful for the polyisocyanates, making it possible to incorporate the polyoxazolidines of the present invention into isocyanate systems without adversely affecting the pot lives thereof. Finally, the process is flexible in that it results in a variety of polyoxazolidines with a wide range of functionalities, generally from 2 to 10.

The polyoxazolidines (I) of the present invention are compatible with most of the common groups of organic polymers. They form latent catalysts in that they hydrolyze instantly in the presence of humidity, even ambient humidity, with opening of the oxazolidine ring and generation of polyalkanol amines. Consequently they are useful as cross-linking agents of e.g. polyisocyanates, polyepoxides and poly(meth)acrylates (Michael addition) in e.g. paint, coating, sealant and adhesive compositions. They are particularly useful in combination with polyisocyanates, in that, owing to their intrinsic characteristics, they do not adversely affect the pot life of the latter and consequently can be combined therewith in one-component systems, which are fluid under normal conditions, with solvents and are cross-linkable with ambient humidity. In these formulations the low viscosity of the polyoxazolidines is particularly advantageous.

Particularly suitable polyisocyanates for the above formulations are both the trimers of hexamethylene diisocyanate and isophorone diisocyanate and the trimers obtained by the partial addition of water to diisocyanates in general and particularly to those obtained when starting from aliphatic and/or aromatic diisocyanates and polyols and/or low molecular weight (around 500 - 20,000) organic difunctional or polyfunctional polymers with (terminal) hydroxyl functionalities. Examples of said polymers are polyesters, polyethers, polycarbonate copolyethers and polycarbonates, polybutadienes and some hybrid polymers such as polycarbonate copolyesters, with terminal hydroxy groups.

The polyisocyanates may be used in combination with the polyoxazolidines of the present invention in amounts such that two equivalents of isocyanate groups in the polyisocyanate correspond to about one oxazolidine equivalent in the polyoxazolidine. Variations in this stoichiometry are possible, without excessively affecting the solidity of the cross-linked products, provided that the polyoxazolidine is preferably present in quantitites of from 30% in defect to 10% in excess with respect to the stoichiometric ratio.

The formulation of polyisocyanates and polyoxazolidines is usually carried out within a temperature range of from room temperature to 60°C, and is facilitated by the perfect compatibility of the two products involved. Catalysts suitable for accelerating the cross-linking may be present in the formulation. Usually said catalysts are selected from metallic soaps, in particular organometallic compounds of tin, and from organic acids, in particular p-toluene sulphonic acid, n-naphthoic acid etc. Besides catalysts, other additives may be incorporated, such as organic or inorganic charges, thixotropic agents, flame-retardants, adhesion promoters, stabilizers and UV-absorbers, depending on the specific use intended.

The formulations thus obtained cross-link at a high rate, owing to the atmospheric humidity, to result in end-products having excellent characteristics, especially with respect to thermal and chemical resistance and resistance to photo-oxidative aging.

The following examples are to further illustrate the invention without limiting the scope thereof.

### EXAMPLE 1 (Comparative)

Preparation of a product of formula 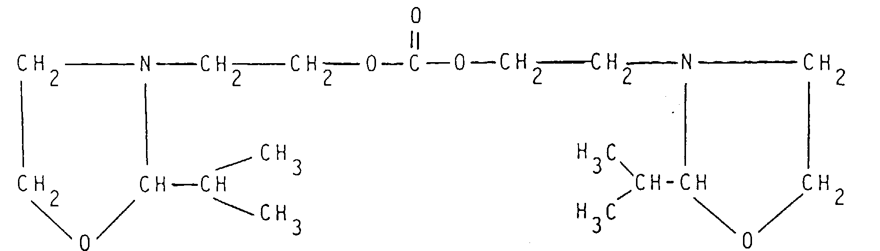 3-(2-Hydroxyethyl)-2-isopropyl-1,3-oxazolidine (580.65 g; 3.6519 moles); diallyl carbonate (DAC) (235.9 g; 1.6599 moles) and sodium methylate in CH₃OH (3 ml of a 30% solution by weight) were placed, under a nitrogen flow, into a flask equipped with magnetic bar stirrer and distillation column with 5 theoretical plates and nitrogen inlet.

The transesterification was carried out at +120°C, progressively reducing the pressure to 30 torr over 4 hours, whereafter allyl alcohol was distilled off (180 g; 3.108 moles; 84% of the theoretical value).

The vacuum was increased to 0.5 torr and the excess hydroxyethyl oxazolidine was eliminated, the temperature of the distillation column being raised from +25 to +100°C. The hydroxyethyl oxazolidine was thus recovered within 3 hours. The distillation temperature of the hydroxyethyl oxazolidine was +76°C at the head of the column (878 g; 0.49 moles).

The hot product was cooled, treated with p-toluene sulphonic acid (3.8 g; 0.195 moles) in methylethylketone (10 ml) for 30 min. at room temperature and finally treated with calcium oxide (15 g) for 60 min.

After filtration, the product had the following elemental analysis: C = 57.35%, H = 9.90%; N = 7.98% (calculated for C₁₇H₃₂O₅H₂, M.W. = 334: C = 58.3%; H = 9.3%; N = 8.14%).

The product had a viscosity of 35 to 40 cps (+25°C), was transparent and orange in colour, sulphur and free acidity being absent therein.

The yield was 550 g, 1.599 moles, corresponding to 96.3% yield based on DAC. The structural formula was confirmed by ¹H-NMR.

The IR spectrum of the product showed a peak at 1740 cm⁻¹ (corresponding to the carbonate bridge) and no peak due to residual allylic groups (1665 cm⁻¹).

### EXAMPLE 2

Preparation of a product of formula

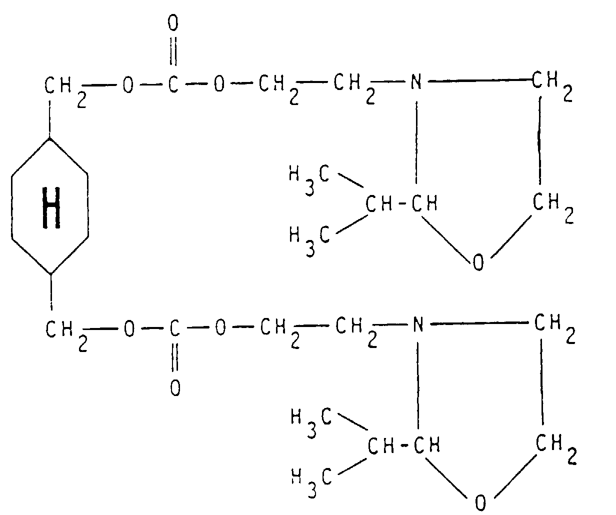

The same procedure as described in example 1 was used, starting with the specified hydroxyethyl oxazolidine (349.8 g, 2.2 moles), cyclohexane dimethanol (144.2 g, 1.0 moles), diallyl carbonate (284.24 g, 2.0 moles) and sodium methylate (2 ml, 30% solution in methanol).

The light distillate composed of allyl alcohol (95%) amounted to 242.1 g (3.98 moles, 99% of the theoretical value).

The hydroxyethyl oxazolidine recovered amounted to 58.5 g (0.36 moles).

The yield of the product represented by the above formula was 472.8 g (0.918 moles, 91.8% yield).

The elemental analysis was C = 60.36%, H = 9.14%, N = 5.1% (calculated for C₂₆H₄₆O₈N₂, M.W. = 514: C = 60.7%, H = 8.95%, N = 5.45%).

The product was treated with p-toluene sulphonic acid for neutralization and finally with CaO, exactly as described in example 1. Filtration over Celite at +70°C eliminated any free acidity (sulphur 1 ppm). The product tended to become opalescent.

The IR spectrum of the product showed a single peak at 1745 cm⁻¹ (corresponding to the structure

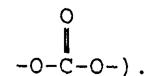

The ¹H-NMR-analysis of the product confirmed the proposed structure.

### EXAMPLE 3

Preparation of product of formula

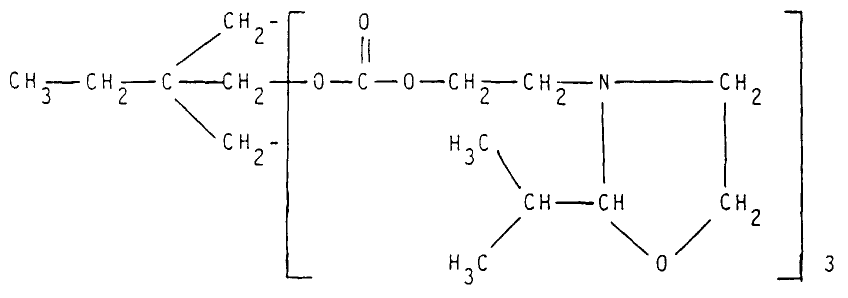

Using the same equipment and procedure as described in example 1, trimethylol propane (TMP) (134.5 g; 1 mole), the hydroxethyl oxazolidine specified in example 1 (524.7 g; 3.3 moles) and diallyl carbonate were placed into the flask.

The transesterification was carried out under the temperature and pressure conditions specified in example 1 and in the presence of sodium methylate (2 ml of a 30% solution by weight in methanol). The subsequent treatment to eliminate the excess, unreacted oxazolidine was the same as that described in example 1.

680 g (0.987 moles) of the desired product were obtained (98.7% yield).

The elemental analysis gave C = 56.95%, H = 9.48%, N = 6.1% (calculated for C₃₃H₅₉H₃O₁₂, M.W. = 689: C = 57.47%, H = 8.56%, N = 6.1%).

The product was in the form of a homogeneous liquid with a viscosity of 2720 cps at +25°C.

The catalyst was eliminated by using p-toluene sulphonic acid and then Cao and subsequent filtration at +100°C over Celite.

The IR spectrum showed the usual peak at 1746 cm⁻¹ and ¹H-NMR analysis confirmed the proposed structure.

### EXAMPLE 4

Preparation of a product of formula

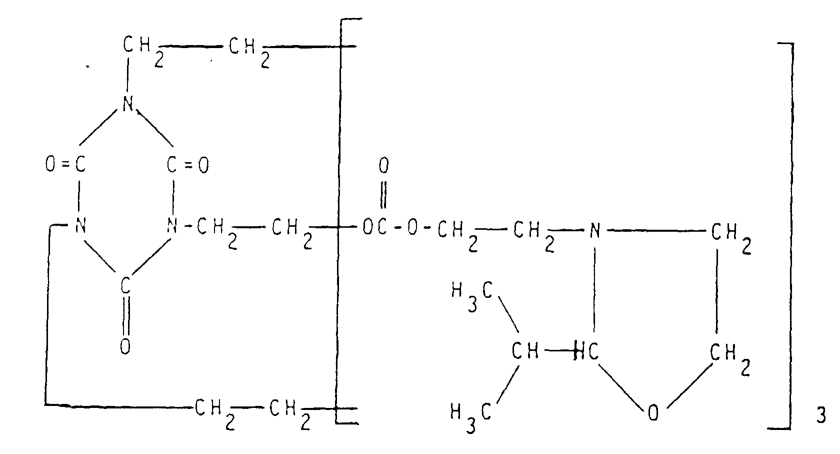

Operating under the same conditions and using the same procedure as in example 1, the transesterification was carried out with trihydroxyethyl isocyanurate (265 g; 1 mole), the hydroxyethyl oxazolidine specified above (511 g; 3.025 moles) and diallyl carbonate (433 g; 3.041 moles) in the presence of sodium methylate (30% in CH₃OH, 3 ml). At the end of the reaction (after 8 hours), 362 g of light distillate composed of 15 g of the corresponding dihydroxyethyl oxazolidine were obtained, whereas the desired reaction product remained in the flask and was treated first with p-toluene sulphonic acid (3.6 g in 10 ml of methylethylketone) and then with Cao (15 g) and was finally filtered.

The resulting product amounted to 738 g (97.8% of the theoretical value) and was in the form of a limpid, transparent liquid whose elemental analysis gave the following results: C = 52.4%, H = 7.68%; N = 9.93% (calculated for C₃₆H₆₀N₆O₁₅, M.W. = 816: C = 52.94%, H = 7.35%, N = 10.29%).

The ¹H-NMR analysis confirmed the proposed structure, whereas the IR spectrum showed two carbonylic peaks at 1700 and 1750 cm⁻¹, respectively due to the CO groups in the isocyanurate ring and the carbonate bridge.

### EXAMPLE 5 (Comparative)

Preparation of the same product as example 1, starting from dimethyl carbonate instead of diallyl carbonate.

The hydroxyethyl oxazolidine specified above (1408 g; 8.85 moles), dimethyl carbonate (1185 g; 13.16 moles) and sodium methylate (30% alcohol solution by weight, 6 ml, 0.0333 moles) were charged into the reactor of example 1.

The transesterification was carried out at an internal temperature of +115°C at atmospheric pressure, then under reduced pressure and finally under maximum vacuum (0.1 torr) for a total period of 10 hours.

During the distillation phase at atmospheric pressure, 306 g, and in the reduced pressure phase 814 g of distillate were recovered, giving a total amount of 1120 g. According to gas chromatography, th e product consisted of methanol (278 g, 97.8% of the theoretical value) and DMC (788 g, 100% of the theoretical value). The hot product amounted to 1467 g (4.26 moles; 96.4% yield).

The hot product, cooled to room temperature, was neutralized by slowly adding, dropwise and under stirring, p-toluenesulphonic acid monohydrate (6.28 g; 0.033 moles) dissolved in MEK (50 ml). After stirring for 1 hour, CaO (20 g) was added in powder form, together with anhydrous MgSO₄ (20 g) and freshly distilled isobutyraldehyde (29 ml, 22.5 g). After stirring for 4 hours at room temperature, the product was filtered over a Celite bed. The yield was 1529 g, 95.9% of which consisted of the above product which had the following characteristics:

| | |
|---|---|
| colour: | pale yellow |
| appearance: | limpid, transparent |
| density: | 1.04 g/cm³ (+20°C) |
| viscosity: | 33 cps (+25°C) |

and the following elemental analysis: C = 58.52%, H = 9.59%, N = 7.37%; S absent, calculated purity 95%.

### EXAMPLE 6

Use of the polyoxazolidines obtained in examples 1-5 in the formulation of sealants.

Prepolymers having -NCO end groups were used in two different types of plasticizer, i.e. the commercial product MESAMOL® (phenolalkyl sulphonate) and benzyl butyl phthalate, resp. (see in Table 1 % weight of plasticizer of the total composition).

The prepolymers having -NCO end groups were obtained by reacting (a) the commercial product RAVECARB® 107 (Enichem Synthesis) which is a liquid polycarbonate having terminal hydroxy groups of formula: 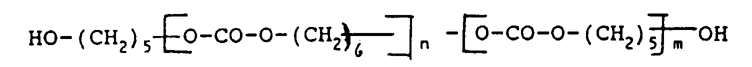 wherein the two repeating units are distributed at random, n and m are integers such that the molecular weight of the product is 1850 whereas the hydroxyl number is 64 (expressed as mg of KOH/g of polycarbonate); or a copolyester polycarbonate (RAVECARB® 111 of Enimont Synthesis) having a number average molecular weight of 2000 and an OH number of 56 (expressed as above); or a commercial polycarbonate diol of hexanediol (RAVECARB® 102 of Enichem Synthesis) having a number average molecular weight of 1000 and an OH number of 112 (expressed as above) with (b) isophorone diisocyanate in a -NCO/-OH ratio of 2.05/1.

The prepolymers diluted in the plasticizer specified above were formulated with the polyoxazolidines produced in examples 1 -5 in the proportions indicated in Table 1 and with 350 ppm of dibutyltin dilaurate, in the form of sheets having a thickness of 2 mm.

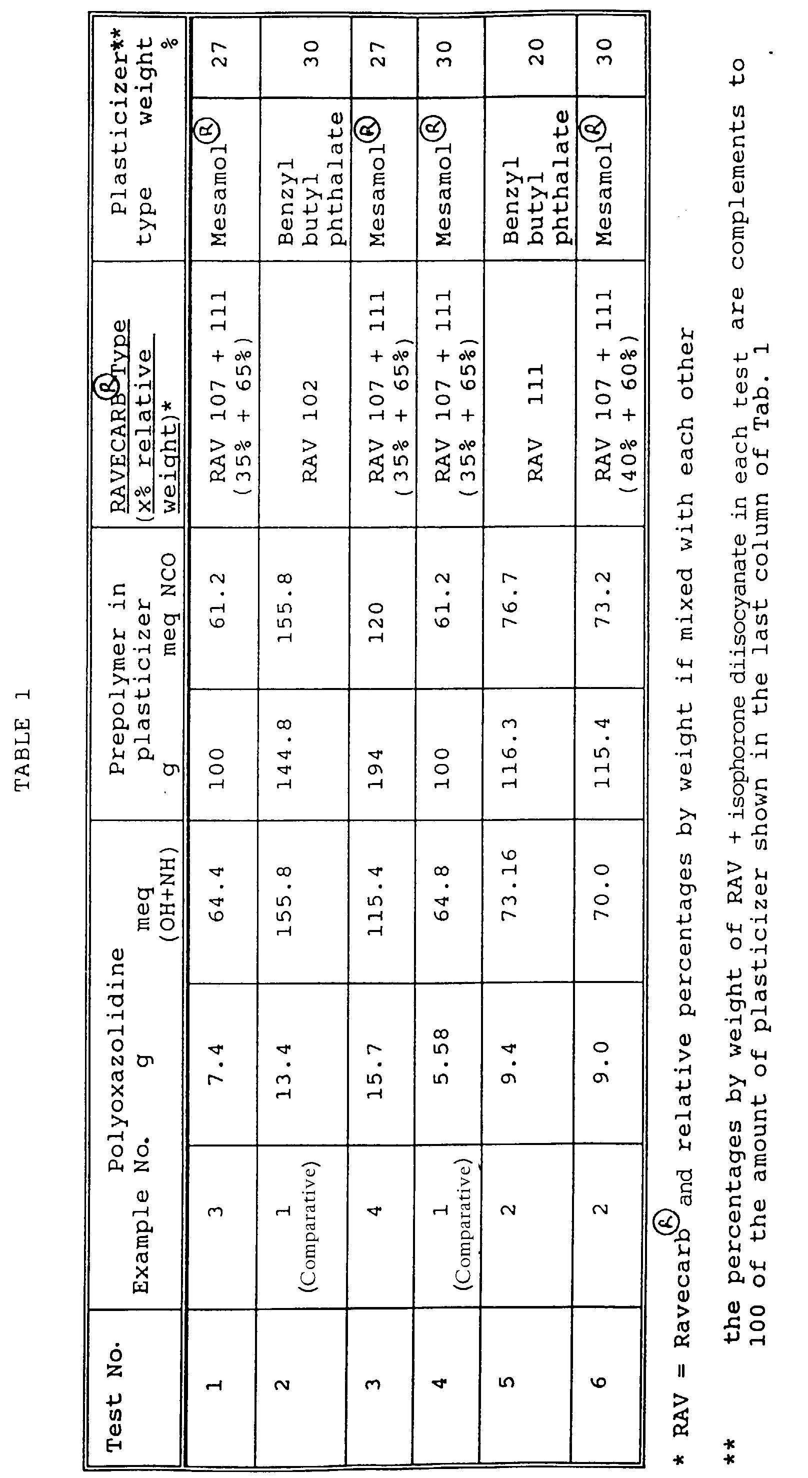

An examination of the sheets obtained, after 30 days of crosslinking at ambient humidity and temperature, gave the results shown in Table 2.

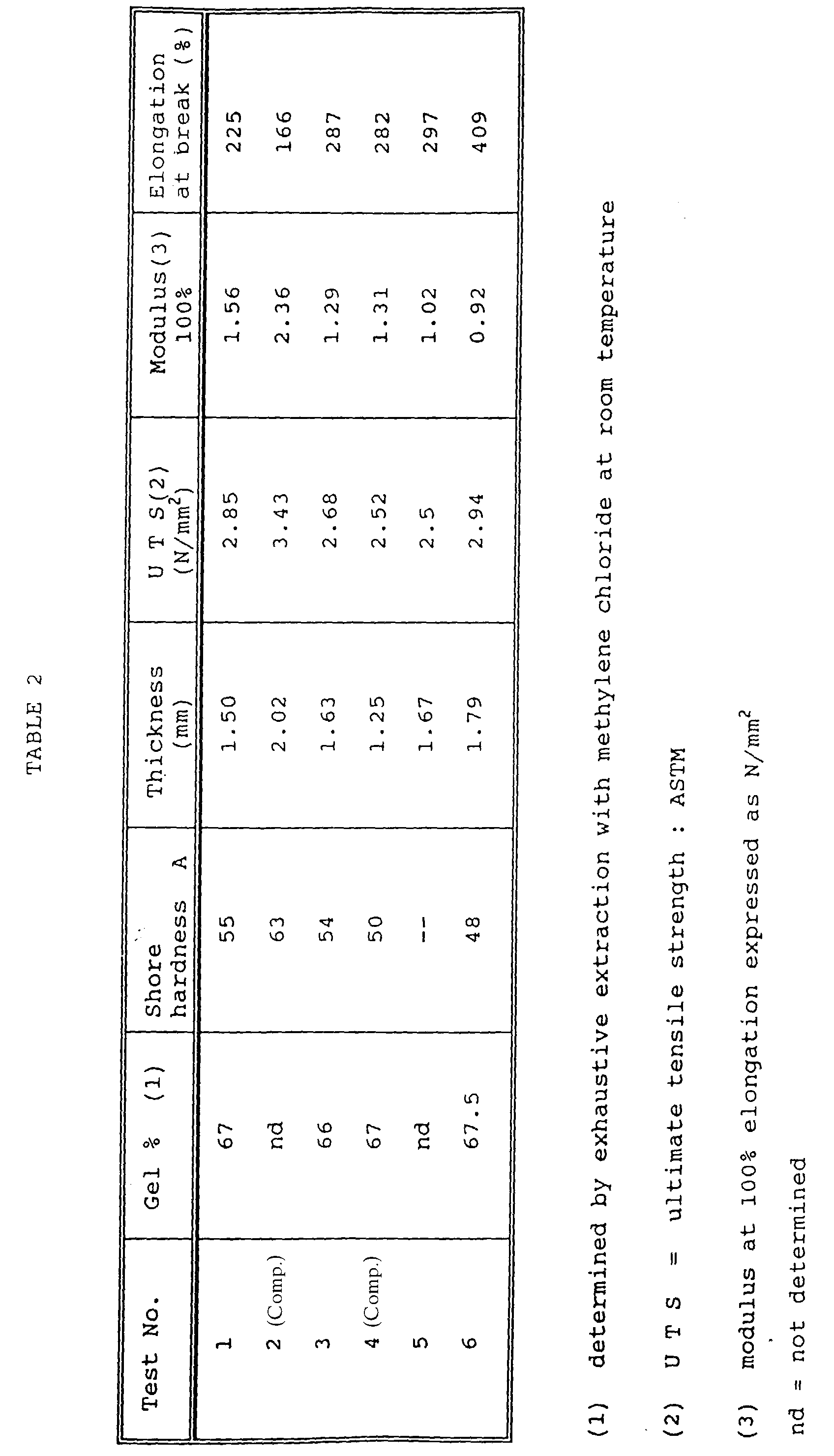

### EXAMPLE 7

The polyoxazolidines prepared in examples 1 - 5 were used in the formulation of paints with the addition of the commercial product DESMODUR® N75 (Bayer; 75% by weight of the trimer of hexamethylene diisocyanate in xylene).

The relative quantities of the two products are expressed in MEQ (milliequivalents; 1 equivalent of polyoxazolidine corresponds to M/2n where M is the average molecular weight of the polyoxazolidine whereas n has the meaning given above; for example, n = 2 for the polyoxazolidines of examples 1 and 2 whereas n = 3 for the polyoxazolidines of examples 3 and 4).

The compositions of the individual formulations are shown in Table 3, as well as compositions of those diluted with xylene in the quantities indicated in the same Table.

**TABLE 3**

| Applications of polyoxazolidines of examples 1-5 in paints | | | | | |
|---|---|---|---|---|---|
| TEST NO. | POLYOXAZOLIDINE | | | DESMODUR® N75 | XYLENE ml |
| | EX. NO. | g | MEQ | MEQ NCO | |
| 1 (Comp.) | 1 | 9.03 | 105 | 119 | 150 |
| 2 (Comp.) | 1 | 7.7 | 59.5 | 85.7 | 150 |
| 3 | 3 | 9.93 | 115.9 | 92.7 | 180 |

| COMPARISON | | | | | |
|---|---|---|---|---|---|
| 4 (*) | 9 | 9.63 | 86.7 | 80.7 | 150 |

| | | | | | |
|---|---|---|---|---|---|
| (*) The polyoxazolidine used as comparison was a polyoxazolidine containing polyester groups instead of polycarbonate groups and was prepared as described in example 9 below. | | | | | |

The polyoxazolidines formulated as in Table 3 were in the form of sheets having a thickness of 0.1-0.2 mm for the evaporation of the xylene solvent, and the sheets, when examined after a week of cross-linking under ambient conditions, gave the results shown in Table 4.

The sheets, after having been subjected to dynamo-mechanical characterization, were immersed in deionized water and subjected to hydrolysis in an oven at +65°C.

After two months of aging the polymeric films were removed from the aqueous bath and, after drying and reconditioning, were subjected to dynamo-mechanical analysis. Whereas all of the films based on oxazolidines having a carbonate structure were transparent, the films based on polyester were irreversibly opaque even after a short period of immersion in water. The dynamo-mechanical test results of Table 5 confirm the superiority of the polyoxazolidines having a polycarbonate structure over those having a polyester structure, despite the initially more favourable results obtained with the latter.

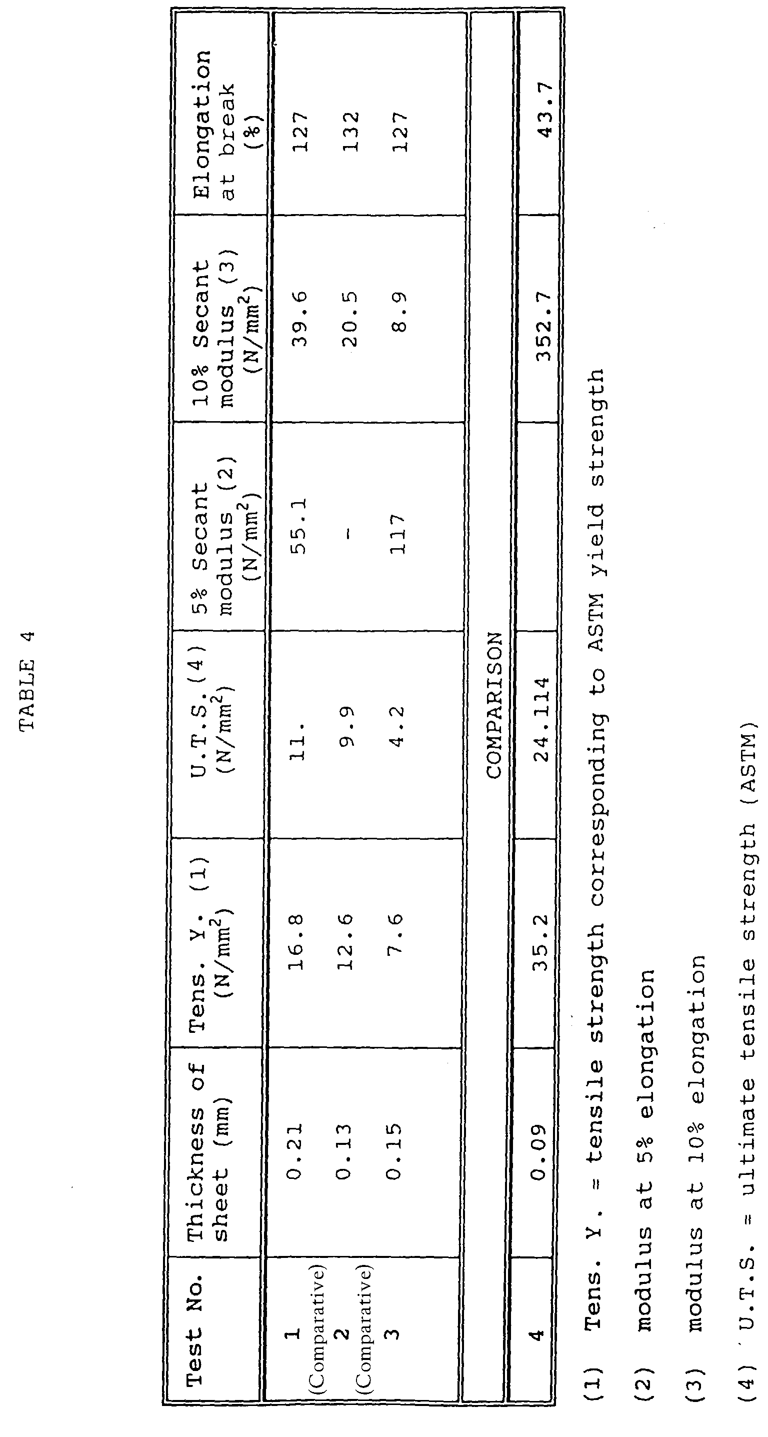

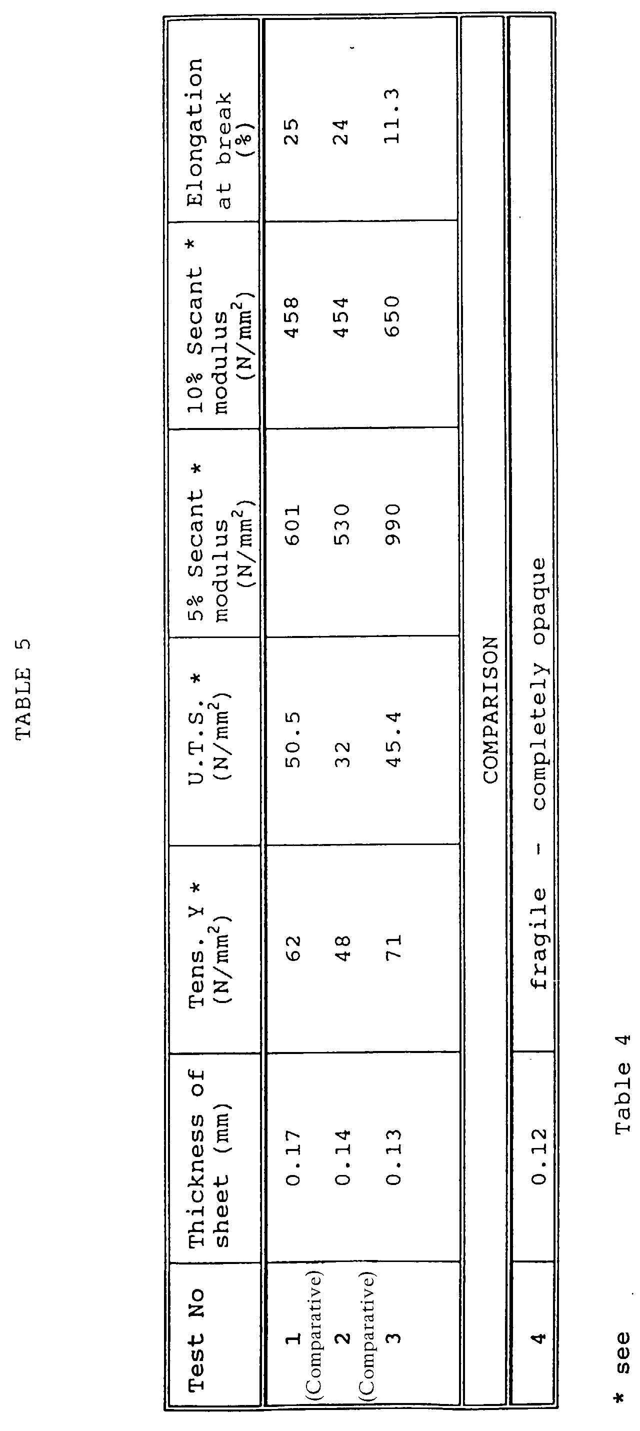

### EXAMPLE 9

The comparison of the polyoxazolidines of the previous examples was carried out by using a polyester groups containing polyoxazolidine of the following formula:

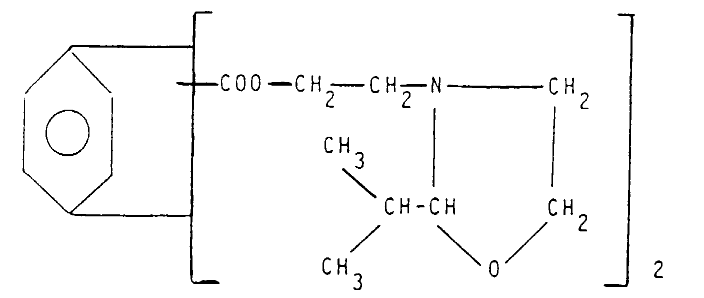

Dimethyl terephthalate (194.19 g, 1 mole), the hydroxyethyl oxazolidine specified above (349.8 g, 2.2 moles) and sodium methylate (2.5 ml of a 30% solution by weight in methanol) were placed into the reactor of example 1.

The temperature was raised to 105 - 110°C and the pressure in the reactor was reduced to 0.5 mm Hg within a period of one hour. The reaction was continued for a further 6 hours, keeping the vacuum and temperature values constant and recovering a total of 64 g (100% of the theoretical value) of methanol and 18.6 g (58.5% of the theoretical value) of hydroxyethyl oxazolidine.

The product was cooled, the catalyst eliminated by adding p-toluene sulphonic acid and the product was then treated with CaO (10 g), heated (105°C), and filtered at that temperature. The yield was 449 g (100%).

Elemental analysis: C = 64.08%; H = 8.16%, N = 6.22% (calculated for C₂₄H₃₂O₆N₂, MW = 444: C = 64.86%, H = 7.21%, N = 6.31%); viscosity: 1100 cps at +25°C. The ¹H-NMR spectrum was in accordance with the proposed structure. The product crystallized at room temperature.

## Claims (Claims for the following Contracting State(s): AT, BE, CH, DE, DK, FR, GB, IT, LI, LU, NL, SE)

1. Polyoxazolidines of general formula (I): 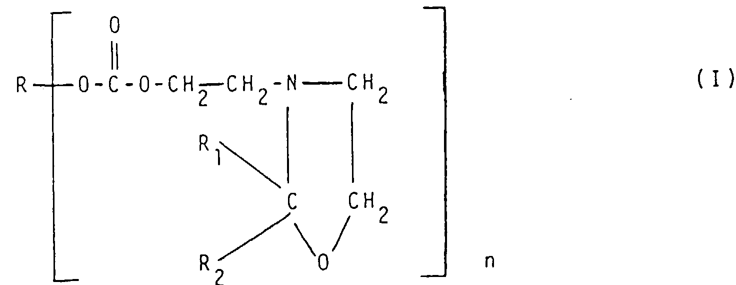 wherein
n ranges from 2 to 10;
R is an n-valent radical selected from:
the trivalent triethylisocyanurate group of formula 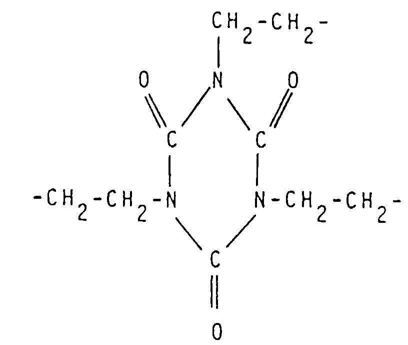 n-valent linear or branched C₂-C₁₂ alkylene groups, C₅-C₁₂ cycloalkylene groups, polyetheneglycol groups of formula -(CH₂-CH₂-O)ₘ-CH₂-CH₂-, m ranging from 1 to 10, the para-dimethylene cyclohexane group of formula 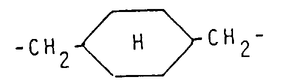 and n-valent groups of general formula 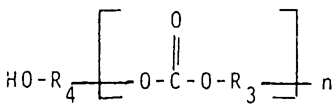 wherein R₃ is the linear, cyclic or aromatic hydrocarbon residue of a glycol containing up to 12 carbon atoms and R₄ has the same meanings as R provided that it is not an N-ethyl-oxazolidine group;
R₁ and R₂, the same or different from each other, represent hydrogen, linear or branched C₁-C₆ alkyl, cycloalkyl or aryl or
R₁ and R₂, together with the carbon atom to which they are linked, form a saturated C₅-C₇ cycloalkylene ring.

2. Polyoxazolidines according to claim 1, wherein R represents a linear or branched bi-, tri- or tetra-valent C₂-C₆ alkylene or a cycloalkylene group.

3. Polyoxazolidines according to any one of claims 1 and 2, wherein R₁ and R₂ independently represent linear or branched C₁-C₄ alkyl, or one of R₁ and R₂ is hydrogen and the other one represents a linear or branched C₁-C₄ alkyl group.

4. Polyoxazolidines according to any one of claims 1 to 3, wherein R₁ and R₂ are selected from the combinations H/H, H/isopropyl, methyl/methyl and methyl/ethyl or together represent a group of formula 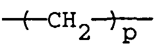 wherein p is 4 or 5.

5. Polyoxazolidines according to any one of claims 1 to 4, wherein R is selected from groups of formulae 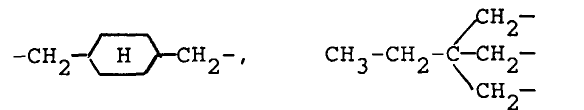 2-(2-isopropyl-1,3-oxazolin-3-yl)ethyl and the trivalent triethylisocyanurate group specified in claim 1.

6. Polyoxazolidines according to any one of claims 1 to 5, wherein n ranges from 2 to 4.

7. Process for the preparation of polyoxazolidines according to any one of claims 1 to 6 wherein
(a) an aldehyde or a ketone of general formula (II): 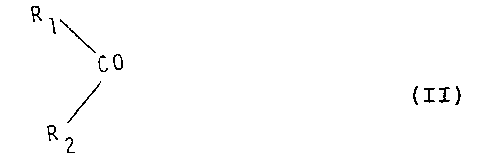 is reacted with diethanolamine of formula (III): 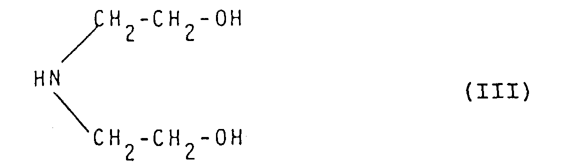 to afford a hydroxyethyl oxazolidine of general formula (IV): 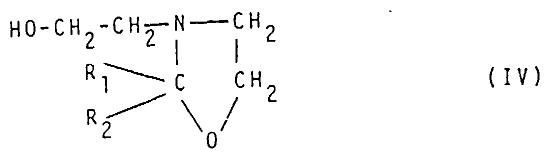 and
(b) said hydroxyethyl oxazolidine (IV) is reacted with a carbonate of general formula (V) and an alcohol of general formula (VI): 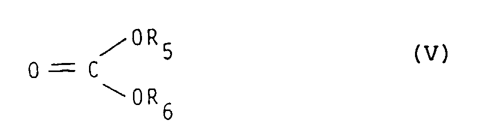
R(-OH)ₙ (VI)
to obtain the polyoxazolidine (I);
R, R₁, R₂ and n having the meanings specified in claims 1 and 2 and
R₅ and R₆ representing saturated or unsaturated C₁-C₄ hydrocarbon groups or aryl groups or together with the two adjacent oxygen atoms and the carbonyl carbon atom forming a cyclic carbonate of general formula 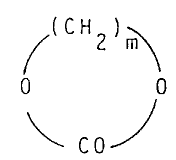 wherein m is 2 or 3.

8. Process according to claim 7, wherein said carbonate (V) is diallyl carbonate or dimethyl carbonate.

9. Process according to any one of claims 7 and 8, wherein p-toluene sulphonic acid is used as catalyst for step (a).

10. Use of the polyoxazolidines according to any one of claims 1 to 7 as cross-linking agents, particularly for coating, paint, sealant or adhesive compositions based on polyisocyanates, poly(meth)acrylates or polyepoxides.

## Claims (Claims for the following Contracting State(s): ES, GR)

1. Process for the preparation of polyoxazolidines of general formula (I): 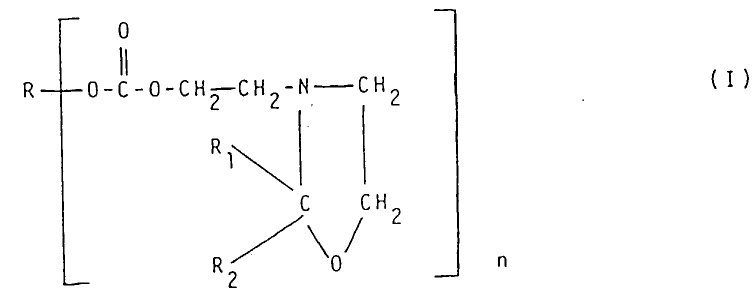 wherein
n ranges from 2 to 10;
R is an n-valent radical selected from:
the trivalent triethylisocyanurate group of formula 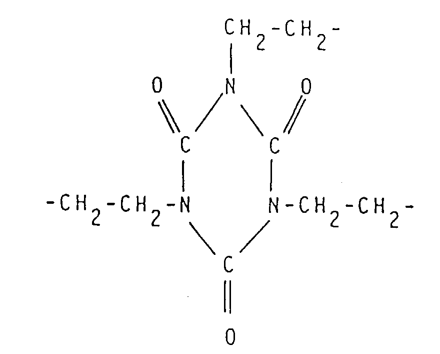 n-valent linear or branched C₂-C₁₂ alkylene groups, C₅-C₁₂ cycloalkylene groups, polyetheneglycol groups of formula -(CH₂-CH₂-O)ₘ-CH₂-CH₂-, m ranging from 1 to 10, the para-dimethylene cyclohexane group of formula 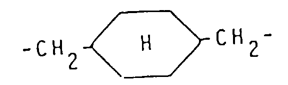 and n-valent groups of general formula 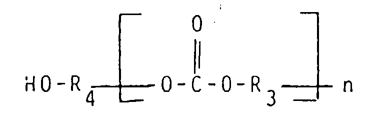 wherein R₃ is the linear, cyclic or aromatic hydrocarbon residue of a glycol containing up to 12 carbon atoms and R₄ has the same meanings as R provided that it is not an N-ethyl-oxazolidine group;
R₁ and R₂, the same or different from each other, represent hydrogen, linear or branched C₁-C₆ alkyl, cycloalkyl or aryl or
R₁ and R₂, together with the carbon atom to which they are linked, form a saturated C₅-C₇ cycloalkylene ring wherein
(a) an aldehyde or a ketone of general formula (II): 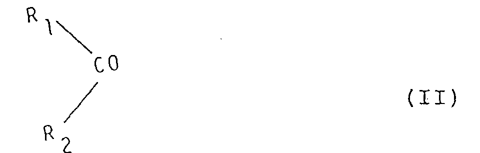 is reacted with diethanolamine of formula (III): 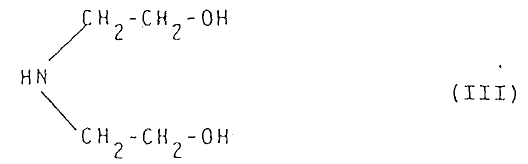 to afford a hydroxyethyl oxazolidine of general formula (IV): 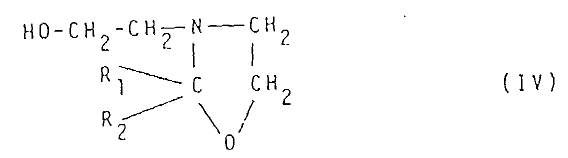 and
(b) said hydroxyethyl oxazolidine (IV) is reacted with a carbonate of general formula (V) and an alcohol of general formula (VI): 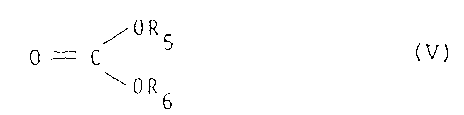
R(-OH)ₙ (VI)
to obtain the polyoxazolidine (I);
R, R₁, R₂ and n having the meanings specified above and
R₅ and R₆ representing saturated or unsaturated C₁-C₄ hydrocarbon groups or aryl groups or together with the two adjacent oxygen atoms and the carbonyl carbon atom forming a cyclic carbonate of general formula 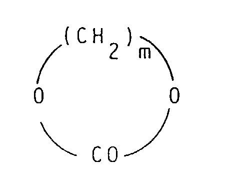 wherein m is 2 or 3.

2. Process according to claim 1, wherein R in formula (I) represents a linear or branched bi-, tri- or tetra-valent C_{**2**}-c_{**6**} alkylene or a cycloalkylene group.

3. Process according to any one of claims 1 and 2, wherein R_{**1**} and R_{**2**} in formula (I) independently represent linear or branched C_{**1**}-C_{**4**} alkyl, or one of R_{**1**} and R_{**2**} is hydrogen and the other one represents a linear or branched C_{**1**}-C_{**4**} alkyl group.

4. Process according to any one of claims 1 to 3, wherein R_{**1**} and R_{**2**} in formula (I) are selected from the combinations H/H. H/isopropyl, methyl/methyl and methyl/ethyl or together represent a group of formula 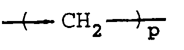 wherein p is 4 or 5.

5. Process according to any one of claims 1 to 4, wherein R in formula (I) is selected from groups of formulae 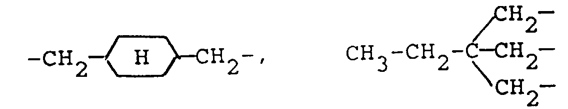 2- (2-isopropyl-1,3-oxazolin-3-yl)ethyl and the trivalent triethylisocyanurate group specified in claim 1.

6. Process according to any one of claims 1 to 5, wherein n in formula (I) ranges from 2 to 4.

7. Process according to any one of claims 1 to 6, wherein said carbonate (V) is diallyl carbonate or dimethyl carbonate.

8. Process according to any one of claims 1 to 7, wherein p-toluene sulphonic acid is used as catalyst for step (a).

9. Process for cross-linking organic polymers wherein polyoxazolidines prepared according to the process of any one of claims 1 to 8, are used as cross-linking agents, particularly for coating, paint, sealant or adhesive compositions based on polyisocyanates, poly(meth)acrylates or polyepoxides.

## Patentansprüche (Patentansprüche für folgende(n) Vertragsstaat(en): AT, BE, CH, DE, DK, FR, GB, IT, LI, LU, NL, SE)

1. Polyoxazolidine der allgemeinen Formel (I): 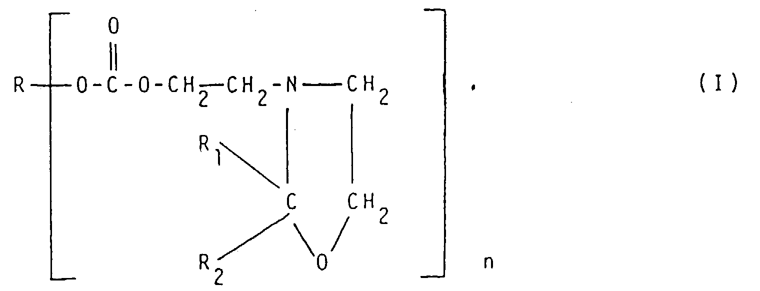 worin:
n = von 2 bis 10 ist:
R = eine n-wertige Gruppe ist, ausgewählt aus:
der dreiwertigen Triethylisocyanuratgruppe der Formel 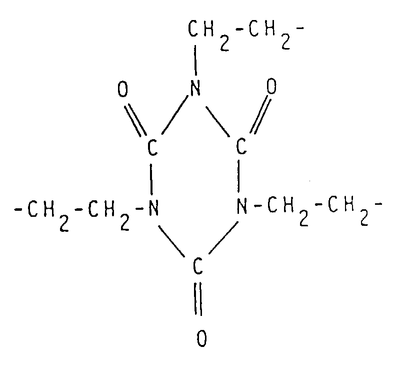 n-wertigen linearen oder verzweigten C_{**2**}-C_{**12**}-Alkylengruppen, C_{**5**}-C_{**12**}-Cycloalkylengruppen, Polyethenglykolgruppen der Formel -(CH₂-CH₂-O)ₘ-CH₂-CH₂-, worin m = von 1 bis 10 ist; der para-Dimethylencyclohexangruppe der Formel 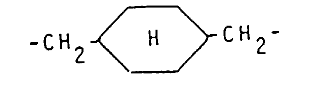 und n-wertigen Gruppen der allgemeinen Formel 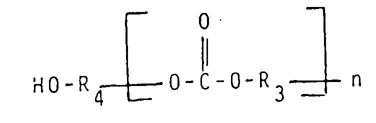 worin R_{**3**} ein linearer, cyclischer oder aromatischer Kohlenwasserstoffrest eines Glykols ist, das bis zu 12 Kohlenstoffatome aufweist, R_{**4**} die gleiche Bedeutung wie R hat unter der Voraussetzung, daß es keine N-Ethyl-oxazolidingruppe ist;
R_{**1**} und R_{**2**}, die gleich oder verschieden sein können, jeweils Wasserstoff, lineares oder verzweigtes C_{**1**}-C_{**6**}Alkyl, Cycloalkyl oder Aryl bedeuten oder
R_{**1**} und R_{**2**} zusammen mit dem Kohlenstoffatom, an das sie gebunden sind, einen gesättigten C_{**5**}-C_{**7**}-Cycloalkylenring bilden.

2. Polyoxazolidine nach Anspruch 1, worin R eine lineare oder verzweigte zwei-, drei- oder vierwertige C_{**2**}-C_{**6**}-Alkylen- oder eine Cycloalkylengruppe bedeuten.

3. Polyoxazolidine nach einem der Ansprüche 1 und 2, worin R_{**1**} und R_{**2**} unabhängig voneinander ein lineares oder verzweigtes C_{**1**}-C_{**4**}-Alkyl bedeuten oder eine der Gruppen R_{**1**} und R_{**2**} Wasserstoff und die andere eine lineare oder verzweigte C_{**1**}-C_{**4**}-Alkylgruppe bedeutet.

4. Polyoxazolidine nach einem der Ansprüche 1 bis 3, worin R_{**1**} und R_{**2**} ausgewählt sind aus den Kombinationen H/H, H/Isopropyl, Methyl/Methyl und Methyl/Ethyl oder zusammen eine Gruppe der Formel 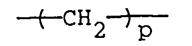 bedeuten, worin p = 4 oder 5 ist.

5. Polyoxazolidine nach einem der Ansprüche 1 bis 4, worin R ausgewählt wird aus Gruppen der Formeln 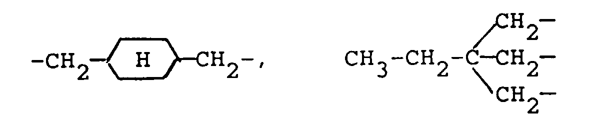 2-(2-Isopropyl-1,3-oxazolin-3-yl) ethyl und der dreiwertigen Triethylisocyanuratgruppe nach Anspruch 1.

6. Polyoxazolidine nach einem der Ansprüche 1 bis 5, worin n von 2 bis 4 ist.

7. Verfahren zur Herstellung von Polyoxazolidinen nach einem der Ansprüche 1 bis 6, worin
(a) ein Aldehvd oder ein Keton der allgemeinen Formel (II): 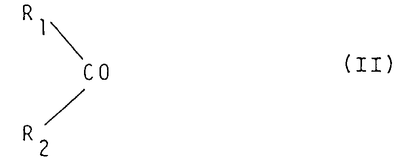 umgesetzt wird mit Diethanolamin der Formel (III): 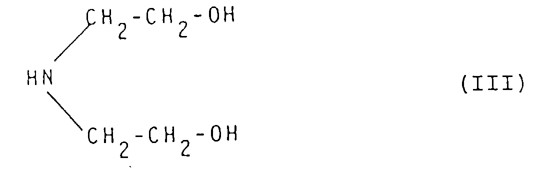 um ein Hydroxyethyloxazolidin der allgemeinen Formel (IV) zu ergeben: 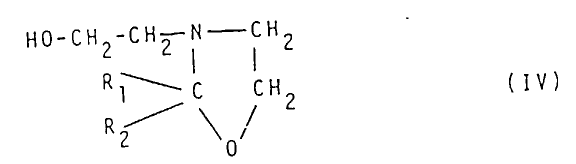 und
(b) das genannte Hydroxyethyloxazolidin (IV) umgesetzt wird mit einem Carbonat der allgemeinen Formel (V) und einem Alkohol der allgemeinen Formel (VI): 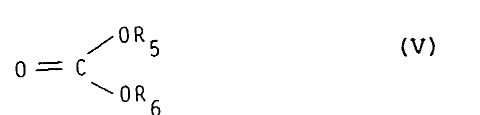
R(-OH)ₙ (VI)
zum Erhalt des Polyoxazolidins (I); wobei
R, R_{**1**}, R_{**2**} und n die in den Ansprüchen 1 und 2 definierten Bedeutungen haben und
R_{**5**} und R_{**6**} gesättigte oder ungesättigte C_{**1**}-C_{**4**}-Kohlenwasserstoffgruppen oder Arylgruppen bedeuten oder zusammen mit den 2 angrenzenden Sauerstoffatomen und dem Carbonylkohlenstoffatom ein cyclisches Carbonat bilden mit der allgemeinen Formel 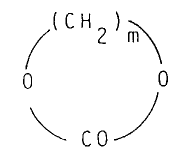 worin m = 2 oder 3 ist.

8. Verfahren nach Anspruch 7, worin das genannte Carbonat (V) Diallylcarbonat oder Dimethylcarbonat ist.

9. Verfahren nach einem der Ansprüche 7 und 8, worin p-Toluolsulfonsäure als Katalysator für den Schritt (a) verwendet wird.

10. Verwendung der Polyoxazolidine nach einem der Ansprüche 1 bis 7 als Vernetzungsmittel, insbesondere für Beschichtungen, Farben, Dichtungsmittel oder Klebstoffzusammensetzungen auf der Basis von Polyisocyanaten, Poly(meth)acrylaten oder Polyepoxiden.

## Patentansprüche (Patentansprüche für folgende(n) Vertragsstaat(en): ES, GR)

1. Verfahren zur Herstellung von Polyoxazolidinen der allgemeinen Formel (I): 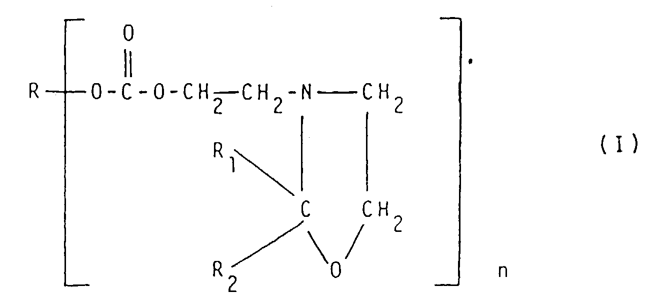 worin:
n = von 2 bis 10 ist:
R = eine n-wertige Gruppe ist, ausgewählt aus:
der dreiwertigen Triethylisocyanuratgruppe der Formel 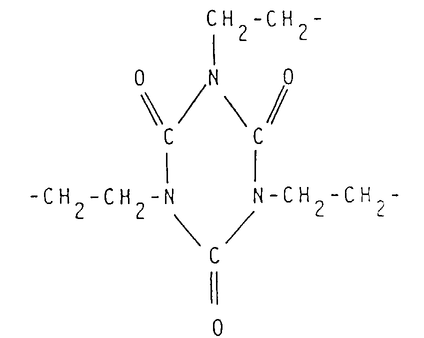 n-wertigen linearen oder verzweigten C_{**2**}-C_{**12**}-Alkylengruppen, C_{**5**}-C_{**12**}-Cycloalkylengruppen, Polyethenglykolgruppen der Formel -(CH₂-CH₂-O)ₘ-CH₂-CH₂-, worin m = von 1 bis 10 ist; der para-Dimethylencyclohexangruppe der Formel 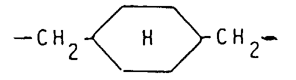 und n-wertigen Gruppen der allgemeinen Formel 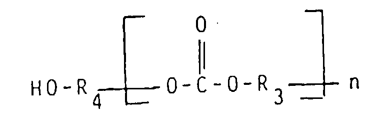 worin R_{**3**} ein linearer, cyclischer oder aromatischer Kohlenwasserstoffrest eines Glykols ist, das bis zu 12 Kohlenstoffatome aufweist, R_{**4**} die gleiche Bedeutung wie R hat unter der Voraussetzung, daß es keine N-Ethyl-oxazolidingruppe ist;
R_{**1**} und R_{**2**}, die gleich oder verschieden sein können, jeweils Wasserstoff, lineares oder verzweigtes C_{**1**}-C_{**6**}-Alkyl, Cycloalkyl oder Aryl bedeuten oder
R_{**1**} und R_{**2**} zusammen mit dem Kohlenstoffatom, an das sie gebunden sind, einen gesättigten C_{**5**}-C_{**7**}-Cycloalkylenring bilden, bei dem
(a) ein Aldehyd oder ein Keton der allgemeinen Formel (II): 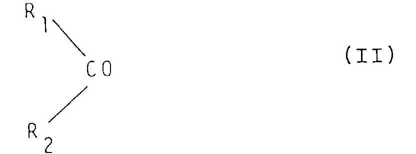 umgesetzt wird mit Diethanolamin der Formel (III): 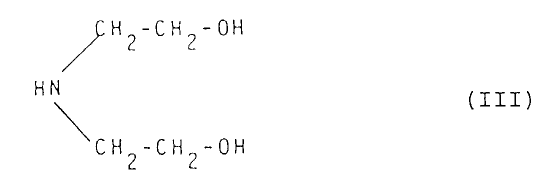 um ein Hydroxyethyloxazolidin der allgemeinen Formel (IV) zu ergeben: 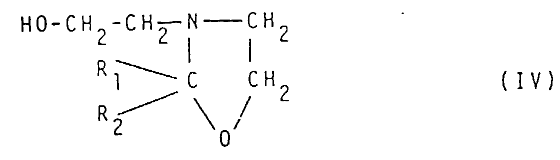 und
(b) das genannte Hydroxyethyloxazolidin (IV) umgesetzt wird mit einem Carbonat der allgemeinen Formel (V) und einem Alkohol der allgemeinen Formel (VI): 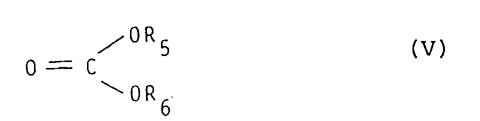
R(-OH)ₙ (VI)
zum Erhalt des Polyoxazolidins (I); wobei
R, R_{**1**}, R_{**2**} und n die oben definierten Bedeutungen haben und R_{**5**} und R_{**6**} gesättigte oder ungesättigte C_{**1**}-C_{**4**}-Kohlenwasserstoffgruppen oder Arylgruppen bedeuten oder zusammen mit den 2 angrenzenden Sauerstoffatomen und dem Carbonylkohlenstoffatom ein cyclisches Carbonat bilden mit der allgemeinen Formel 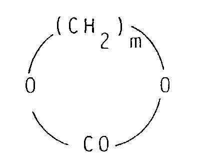 worin m = 2 oder 3 ist.

2. Verfahren nach Anspruch 1, worin R in der Formel (I) eine lineare oder verzweigte zwei-, drei- oder vierwertige C_{**2**}-C_{**6**}-Alkylen-oder eine Cycloalkylengruppe bedeutet.

3. Verfahren nach einem der Ansprüche 1 und 2, worin R_{**1**} und R_{**2**} in der Formel (I) unabhängig voneinander ein lineares oder verzweigtes C_{**1**}-C_{**4**}-Alkyl bedeuten oder eine der Gruppen R_{**1**} und R_{**2**} Wasserstoff und die andere eine lineare oder verzweigte C_{**1**}-C_{**4**}-Alkylgruppe bedeutet.

4. Verfahren nach einem der Ansprüche 1 bis 3, worin R_{**1**} und R_{**2**} in der Formel (I) ausgewählt sind aus den Kombinationen H/H, H/Isopropyl, Methyl/Methyl und Methyl/Ethyl oder zusammen eine Gruppe der Formel 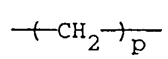 bedeuten, worin p = 4 oder 5 ist.

5. Verfahren nach einem der Ansprüche 1 bis 4, worin R in der Formel (I) ausgewählt wird aus Gruppen der Formeln 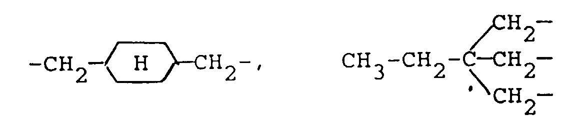 2-(2-Isopropyl-1,3-oxazolin-3-yl)ethyl und der dreiwertigen Triethylisocyanuratgruppe nach Anspruch 1.

6. Verfahren nach einem der Ansprüche 1 bis 5, worin n in der Formel (I) von 2 bis 4 ist.

7. Verfahren nach einem der Ansprüche 1 - 6, worin das genannte Carbonat (V) Diallylcarbonat oder Dimethylcarbonat ist.

8. Verfahren nach einem der Ansprüche 1 bis 7, worin p-Toluolsulfonsäure als Katalysator für den Schritt (a) verwendet wird.

9. Verfahren zum Vernetzen organischer Polymere, worin Polyoxazolidine, hergestellt nach dem Verfahren von einem der Ansprüche 1 bis 8, als Vernetzungsmittel verwendet werden, insbesondere für Überzüge, Farben, Dichtungsmittel oder Haftzusammensetzungen auf der Grundlage von Polyisocyanaten, Poly(meth)acrylaten oder Polyepoxiden.

## Revendications (Revendications pour l'(les) Etat(s) contractant(s) suivant(s): AT, BE, CH, DE, DK, FR, GB, IT, LI, LU, NL, SE)

1. Polyoxazolidines de formule générale (I): 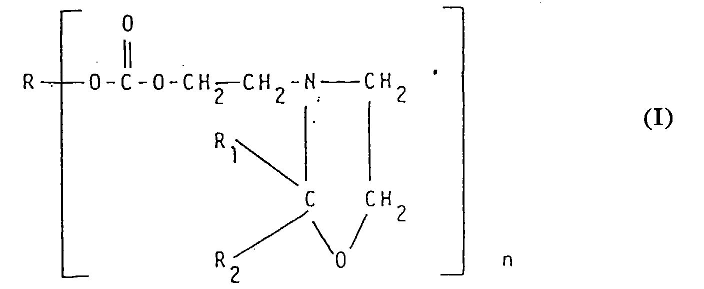 dans laquelle
n est compris entre 2 et 10 ;
R représente un radical de valence n choisi parmi le groupe triéthylisocyanurate trivalent de formule : 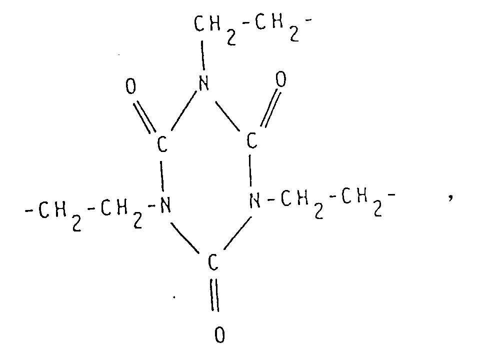 des groupes alkylène en C₂-C₁₂ de valence n, linéaires ou ramifiés, des groupes cycloalkylène en C₅-C₁₂, des groupes polyéthylèneglycol de formule -(CH₂-CH₂-O)ₘ-CH₂-CH₂-, m étant compris entre 1 et 10, le groupe para-diméthylènecyclohexane de formule : 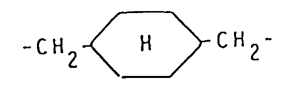 et des groupes de valence n de formule générale : 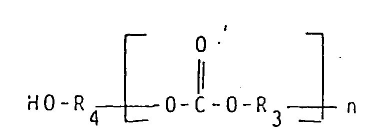 dans laquelle R₃ représente le résidu hydrocarboné linéaire, aromatique ou cyclique, d'un glycol contenant jusqu'à 12 atomes de carbone et R₄ a la même signification que R pourvu qu'il ne représente pas le groupe N-éthyl-oxazolidine ;
R₁ et R₂, identiques ou différents l'un de l'autre, représentent un atome d'hydrogène, un groupe aryle, cycloalkyle ou alkyle en C₁-C₆ ramifié ou linéaire, ou
R₁ et R₂, considérés ensemble avec l'atome de carbone auquel ils sont rattachés, forment un cycle cycloalkylène en C₅-C₇ saturé.

2. Polyoxazolidines conformes à la revendication 1, dans lesquelles R représente un groupe alkylène en C₂-C₆ bi-, tri- ou tétra-valent, linéaire ou ramifié ou un groupe cycloalkylène.

3. Polyoxazolidines conformes à l'une quelconque des revendications 1 et 2, dans lesquelles R₁ et R₂ représentent indépendamment un groupe alkyle en C₁-C₄ ramifié ou linéaire ou, lorsque l'un des symboles R₁ et R₂ représente un atome d'hydrogène, l'autre symbole représente un groupe alkyle en C₁-C₄ linéaire ou ramifié.

4. Polyoxazolidines conformes à l'une quelconque des revendications 1 à 3, dans lesquelles R₁ et R₂ sont choisis parmi les combinaisons H/H, H/isopropyl, méthyl/méthyl et méthyl/éthyl ou, si on les considère ensemble, représentent un groupe de formule -(CH₂)ₚ- dans laquelle p vaut 4 ou 5.

5. Polyoxazolidines conformes à l'une quelconque des revendications 1 à 4, dans lesquelles R est choisi parmi les groupes de formules : 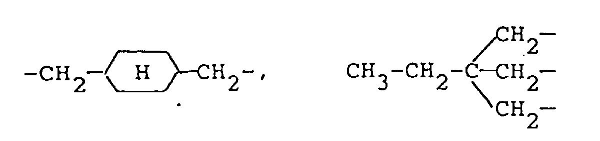 le groupe 2-(2-isopropyl-1,3-oxazolin-3-yl)éthyle et le groupe triéthylisocyanurate trivalent décrit dans la revendication 1.

6. Polyoxazolidines confomes à l'une quelconque des revendications 1 à 5, dans lesquelles n est compris entre 2 et 4.

7. Procédé qui consiste à préparer, les polyoxazolidines conformes à l'une quelconque des revendications 1 à 6 dans lequel :
(a) on fait réagir un aldéhyde ou une cétone de formule générale (II) : 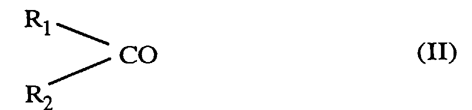 avec la diéthanolamine de formule (III) : 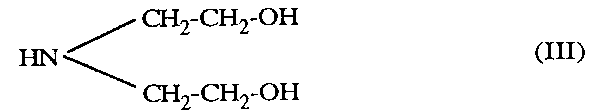 pour obtenir un hydroxyéthyloxazolidine de formule générale (IV) : 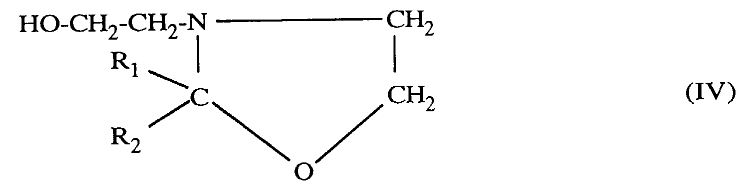 et
(b) on fait réagir ladite hydroxyéthyloxazolidine (IV) avec un carbonate de formule générale (V) et un alcool de formule générale (VI) : 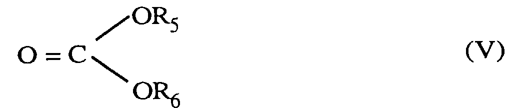
R(-OH)ₙ (VI)
afin d'obtenir la polyoxazolidine (I) ;
R, R₁, R₂ et n ayant les significations indiquées dans les revendications 1 et 2 et,
R₅ et R₆ représentant des groupes hydrocarbonés en C₁-C₄ insaturés ou saturés, ou des groupes aryle ou, si on les considère ensemble avec les deux atomes d'oxygène adjacents et l'atome de carbone du carbonyle, formant un carbonate cyclique de formule générale : 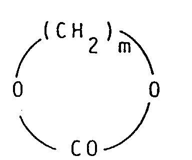 dans laquelle m vaut 2 ou 3.

8. Procédé conforme à la revendication 7, dans lequel ledit carbonate (V) est le carbonate de diallyle ou le carbonate de diméthyle.

9. Procédé conforme à l'une quelconque des revendications 7 et 8, dans lequel on utilise l'acide p-toluène-sulfonique en tant que catalyseur de l'étape (a).

10. Utilisation des polyoxazolidines conformes à l'une quelconque des revendications 1 à 7, en tant qu'agents de réticulation, en particulier pour des compositions de revêtement, de peinture, scellantes ou adhésives à base de polyisocyanates, poly(méth)acrylates ou polyépoxydes.

## Revendications (Revendications pour l'(les) Etat(s) contractant(s) suivant(s): ES, GR)

1. Procédé qui consiste à préparer des polyoxazolidines de formule générale (I): 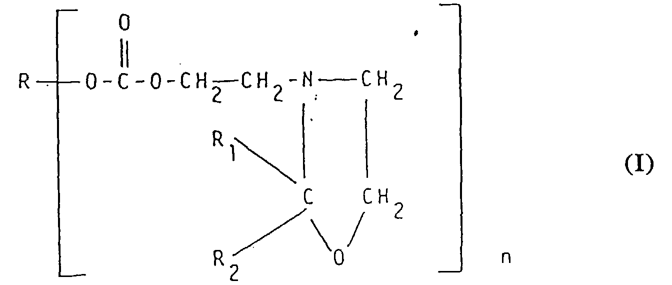 dans laquelle
n est compris entre 2 et 10 ;
R représente un radical de valence n choisi parmi : le groupe triéthyli socyanurate trivalent de formule : 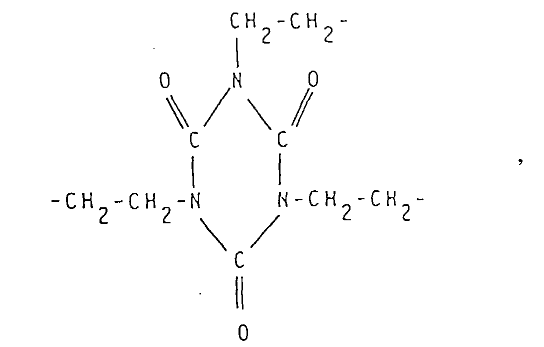 des groupes alkylène en C₂-C₁₂ de valence n, linéaires ou ramifiés, des groupes cycloalkylène en C₅-C₁₂, des groupes polyéthylèneglycol de formule -(CH₂-CH₂-O)ₘ-CH₂-CH₂-, m étant compris entre 1 et 10, le groupe para-diméthylènecyclohexane de formule : 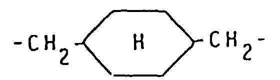 et des groupes de valence n de formule générale : 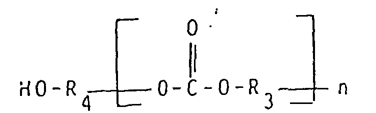 dans laquelle R₃ représente le résidu hydrocarboné linéaire, aromatique ou cyclique, d'un glycol contenant jusqu'à 12 atomes de carbone et
R₄ a la même signification que R pourvu qu'il ne représente pas le groupe N-éthyl-oxazolidine ;
R₁ et R₂, identiques ou différents l'un de l'autre, représentent un atome d'hydrogène, un groupe aryle, cycloalkyle ou alkyle en C₁-C₆ ramifié ou linéaire, ou
R₁ et R₂, considérés ensemble avec l'atome de carbone auquel ils sont rattachés, forment un cycle cycloalkylène en C₅-C₇ saturé ;
procédé dans lequel
(a) on fait réagir un aldéhyde ou une cétone de formule générale (II) : 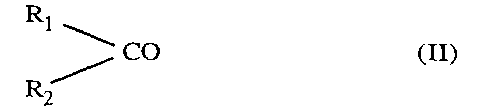 avec la diéthanolamine de formule (III) : 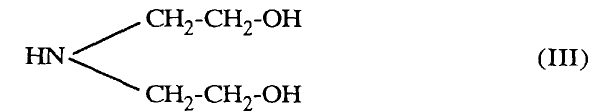 pour obtenir une hydroxyéthyloxazolidine de formule générale (IV) : 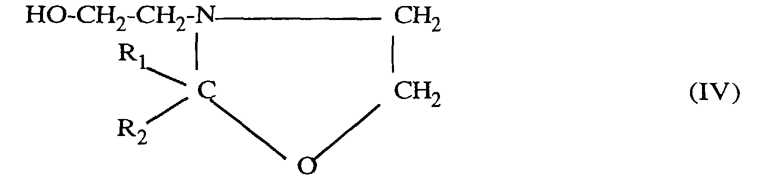 et
(b) on fait réagir ladite hydroxyéthyloxazolidine (IV) avec un carbonate de formule générale (V) et un alcool de formule générale (VI) : 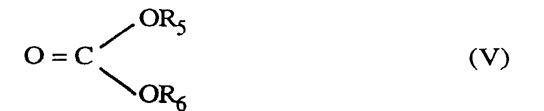
R(-OH)ₙ (VI)
afin d'obtenir la polyoxazolidine (I) ;
R, R₁, R₂ et n ayant les significations indiquées ci-dessus et R₅ et R₆ représentant des groupes hydrocarbonés en C₁-C₄ insaturés ou saturés, ou des groupes aryle ou, si on les considère ensemble avec les deux atomes d'oxygène adjacents et l'atome de carbone du carbonyle, formant un carbonate cyclique de formule générale : 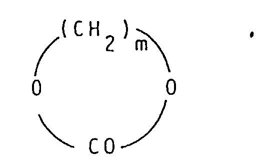 dans laquelle m vaut 2 ou 3.

2. Procédé conforme à la revendication 1, dans lequel R de la formule (I) représente un groupe alkylène en C₂-C₆ bi-, tri- ou tétra-valent, ramifié ou linéaire ou un groupe cycloalkylène.

3. Procédé conforme à l'une quelconque des revendications 1 et 2, dans lequel R₁ et R₂ de la formule (I) représentent indépendamment un groupe alkyle en C₁-C₄ ramifié ou linéaire ou, lorsque l'un des symboles R₁ et R₂ représente un atome d'hydrogène, l'autre symbole représente un groupe alkyle en C₁-C₄ linéaire ou ramifié.

4. Procédé conforme à l'une quelconque des revendications 1 à 3, dans lesquels R₁ et R₂ de la formule (I) sont choisis parmi les combinaisons H/H, H/isopropyl, méthyl/méthyl et méthyl/éthyl ou, si on les considère ensemble, représentent un groupe de formule -(CH₂)ₚ-dans laquelle p vaut 4 ou 5.

5. Procédé conforme à l'une quelconque des revendications 1 à 4, dans lequel R de la formule 1 est choisi parmi les groupes de formules : 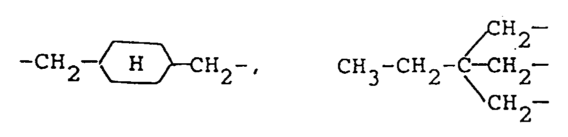 le groupe 2-(2-isopropyl-1,3-oxazolin-3-yl)éthyle et le groupe triéthyl-isocyanurate trivalent décrit dans la revendication 1.

6. Procédé confome à l'une quelconque des revendications 1 à 5, dans lequel n est compris entre 2 et 4.

7. Procédé conforme à l'une quelconque des revendications 1 à 6, dans lequel ledit carbonate (V) est le carbonate de diallyle ou le carbonate de diméthyle.

8. Procédé conforme à l'une quelconque des revendications 1 à 7, dans lequel on utilise l'acide p-toluène-sulfonique en tant que catalyseur de l'étape (a).

9. Procédé qui consiste à faire réticuler des polymères organiques, dans lequel on utilise des polyoxazolidines préparées selon le procédé conforme à l'une quelconque des revendications 1 à 8, en tant qu'agents de réticulation, en particulier pour les compositions de revêtement, de peinture, scellantes ou adhésives à base de polyisocyanates, poly(méth)acrylates ou polyépoxydes.
